# FASCICULE DE BREVET EUROPEEN

(11) **EP 4 138 517 B1**
(45) Date de publication et mention de la délivrance du brevet: **05.03.2025**
(21) Numéro de dépôt: 22200459.0
(22) Date de dépôt: 03.08.2018
(51) Int. Cl.: A61N 5/06, G02B 27/02, H05B 45/10, F21S 6/00, H05B 45/37, F21Y 115/10

(54) **DISPOSITIF D'ECLAIRAGE FACILITANT LA LECTURE**
BELEUCHTUNGSVORRICHTUNG MIT LESEERLEICHTERUNG
LIGHTING DEVICE FACILITATING READING

(30) Priorité: 20.11.2017 FR 1701200
(43) Date de publication de la demande: 22.02.2023
(62) Demande divisionnaire de: 18762368.1
(73) Titulaire: Université de Rennes, 35042 Rennes (FR); Le Floch, Albert, 35700 Rennes (FR)
(72) Inventeur: LE FLOCH, Albert, 35700 Rennes (FR); ROPARS, Guy, 35000 Rennes (FR)
(74) Mandataire: Ex Materia

(56) Documents cités:
- US-A1- 2013 021 386
- ALBERT LE FLOCH ET AL: "Left-right asymmetry of the Maxwell spot centroids in adults without and with dyslexia", BIOLOGICAL SCIENCES, vol. 284, no. 1865, 25 October 2017 (2017-10-25), pages 20171380, XP055490044, ISSN: 0962-8452, DOI: 10.1098/rspb.2017.1380
- ALBERT LE FLOCH ET AL: "Left-right asymmetry of the Maxwell spot centroids in adults without and with dyslexia Eleczronic Supplement Material", 18 October 2017 (2017-10-18), pages 2 - 3, XP055510300, Retrieved from the Internet <URL:http://rspb.royalsocietypublishing.org/highwire/filestream/77828/field_highwire_adjunct_files/0/rspb20171380supp1.pdf> [retrieved on 20180926]
- LA VRAIE DÉMOCRATIE: "Des physiciens de Rennes ont percé le mystère de la dyslexie", YOUTUBE, 19 October 2017 (2017-10-19), pages 2 pp., XP054978716, Retrieved from the Internet <URL:https://www.youtube.com/watch?v=12EoF5YS8Zg> [retrieved on 20180927]

## Description

### 1. Domaine de l'invention.

La présente invention concerne un dispositif d'éclairage. L'invention concerne plus particulièrement un dispositif d'éclairage facilitant la lecture de contenus, notamment textuels, pour des personnes sujettes à la dyslexie.

### 2. Etat de l'art.

La dyslexie est communément définie comme un ensemble de troubles de la lecture qui apparaissent à l'enfance. Il s'agit de troubles spécifiques d'apprentissage dont les causes apparaissent complexes et ont fait et font toujours l'objet de nombreuses études dans des domaines variés.

Il est généralement exclu de considérer que les causes de la dyslexie puissent être uniquement d'ordre sensoriel, social ou psychologique.

Des études menées dans le domaine des neurosciences permettent de penser qu'il pourrait s'agir d'un trouble neurologique spécifique.

Les progrès réalisés dans le domaine de l'imagerie médicale ont pu mettre en évidence le rôle de certaines zones du cerveau dans les processus de lecture et de maîtrise du langage.

Les solutions apportées pour traiter les troubles de la dyslexie s'appuient sur des travaux et activités ludiques selon les difficultés propres à chaque sujet. L'objectif d'un tel accompagnement est d'apporter au sujet objet de troubles une autonomie en matière de lecture. Les méthodes connues sont développées autour de travaux dans des domaines tels que la psychologie, la psychomotricité et l'orthoptie, par exemple.

Récemment, des études ont été conduites, établissant une corrélation entre des particularités propres au mécanisme de la vision et la présence de troubles spécifiques de la dyslexie. La publication "Left-right asymmetry of the Maxwell spot centroids in adults without and with dyslexia (Le Floch A, Ropars G. 2017, Proc. R. Soc. B 284: 20171380, http://dx.doi.org/10.1098/rspb.2017.1380) mentionne le rôle des fovéas, situées dans l'œil humain, dans la construction des images perçues, au niveau cérébral, et le fait que des caractéristiques identiques ou sensiblement identiques pour les deux yeux d'un même sujet entraînent chez lui des dysfonctionnements dans le processus de la vision et du traitement phonologique au niveau cérébral. La transmission d'une image miroir d'un hémisphère à l'autre, par exemple, perturbe sensiblement le processus de lecture d'éléments graphiques ou de contenus textuels chez des sujets présentant des troubles caractéristiques de la dyslexie. Des troubles liés à une instabilité de la fixation et/ou une instabilité posturologique, ou encore des défauts de convergence binoculaire liés aux muscles oculomoteurs, peuvent également entraîner des encombrements visuels comme l'effet miroir.

### 3. Résumé de l'invention.

L'invention permet d'améliorer au moins certains des inconvénients de l'art antérieur en proposant un dispositif d'éclairage adapté à faciliter la lecture de contenus, tels que des contenus graphiques, sur tout type de supports, et notamment sur un support papier. Le dispositif proposé opère des périodes d'activation et de désactivation ou suppression successives d'un faisceau lumineux dans le spectre de la lumière visible. Le faisceau lumineux est ainsi activé et désactivé (supprimé) périodiquement à partir du dispositif d'éclairage selon des cycles successifs opérés à une fréquence prédéterminée comprise dans un intervalle de valeurs allant de 60 à 90 Hz, grâce à un système accordable fonctionnant alors en dispositif anti-encombrement visuel. Ce système met à profit les mécanismes hébbiens dans les neurones du cortex. Les périodes d'activation successives ont chacune une durée comprise dans un intervalle de valeurs allant de 15 à 30% de la durée totale des cycles opérés.

L'invention concerne plus particulièrement un dispositif d'éclairage comprenant une unité de contrôle et un module d'éclairage adapté à la génération d'un faisceau lumineux dans un spectre de lumière visible, caractérisé en ce que ledit dispositif d'éclairage est configuré pour activer et désactiver périodiquement ledit faisceau d'éclairage selon des cycles successifs opérés à une fréquence Fd prédéterminée, chaque cycle ayant une durée T et comprenant une période d'activation du faisceau lumineux de durée T1 précédée ou suivie d'une période de désactivation du faisceau lumineux de durée T2, en ce que la fréquence Fd est comprise dans un intervalle de valeurs allant de 60 à 90 Hz, et en ce que la durée T1 des périodes d'activation du faisceau lumineux est comprise dans un intervalle de valeurs allant de 15 à 30% de la durée T des cycles.

Selon l'invention, il est entendu par « désactivation » du faisceau lumineux, une absence totale d'émission du faisceau lumineux ou encore une émission de puissance bien inférieure au niveau d'émission de la phase d'activation, de sorte que la différence de perception qui en découle induise des effets similaires, sur un sujet dyslexique, aux effets découverts du fait de l'absence de faisceaux selon les cycles précédemment décrits. La désactivation du faisceau lumineux peut donc être totale (le faisceau lumineux est totalement absent après désactivation) ou partielle.

Selon un mode de réalisation de l'invention, la fréquence Fd prédéterminée du faisceau lumineux est définie par un utilisateur du dispositif d'éclairage.

Selon un mode de réalisation de l'invention, la fréquence prédéterminée du faisceau est sélectionnée de façon discrète, c'est-à-dire parmi une pluralité de fréquences prédéterminées dans l'intervalle de fréquences susmentionné.

Selon un mode de réalisation de l'invention, la fréquence Fd varie dans le temps, afin de faciliter plus encore, dans certains cas, l'effacement de l'encombrement visuel et la stabilité binoculaire.

Selon un mode de réalisation de l'invention, la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

Selon un mode de réalisation de l'invention, ladite vitesse de décroissance et égale à ladite vitesse de croissance.

Selon un mode de réalisation de l'invention, lesdits pas successifs sont de durées égales.

Selon un mode de réalisation de l'invention, lesdits pas successifs varient en durée de sorte que la valeur de ladite fréquence Fd évolue selon une forme d'onde en triangle, en scie, ou en sinusoïde entre ladite valeur maximale et ladite valeur minimale.

Selon un mode de réalisation de l'invention, la durée T1 des périodes d'activation du faisceau lumineux varie dans le temps.

Selon un mode de réalisation de l'invention, le faisceau réalisé dans un spectre de lumière visible est généré à partir d'un ou plusieurs éléments de type LED.

Avantageusement, l'utilisation d'une gamme de fréquences à partir de 60 Hz permet de s'affranchir des effets de clignotements perceptibles par l'œil de l'être humain, la limite de perception du clignotement par l'œil se situant aux alentours de 60 Hz, pour l'être humain (hors considération, des espèces animales et des insectes).

Avantageusement, l'alternance de périodes d'activation et de désactivation (ou suppression, ou encore atténuation sensible, ou encore inhibition) du faisceau lumineux, établi dans le spectre de la lumière visible et appliqué à (orienté vers) un support, permet une "focalisation" du cerveau d'un sujet regardant ce support éclairé, sur une image représentative du contenu représenté sur le support observé, puis, une disparition ou quasi-disparition de cette même image de la vue du sujet avant qu'elle ne soit transmise sous forme d'image miroir entre un hémisphère cérébral et l'autre hémisphère cérébral de ce sujet regardant ce support éclairé. Le délai requis pour que le cerveau transmette une image, perçue par l'œil, entre un hémisphère et l'autre hémisphère, sous forme d'image miroir pour ce dernier, est de l'ordre de 10 ms.

Ainsi, le cerveau privilégie l'image transmise par rapport à son image miroir, et la confusion existante chez le sujet qui présente une forte similarité des caractéristiques de ses deux fovéas, est moindre ou sensiblement diminuée pour la lecture du contenu (graphique et/ou textuel) représenté sur le support, notamment lorsque ce contenu est représentatif d'un ou plusieurs contenus textuels.

Selon le mode de réalisation préféré de l'invention, l'utilisation d'un commutateur configuré pour obtenir sélectivement un faisceau lumineux permanent (éclairage ordinaire) et un faisceau lumineux non permanent (éclairage selon l'invention) permet à un utilisateur sujet à des troubles dyslexiques de comparer ses performances usuelles en lecture (obtenues avec un éclairage ordinaire) à ses performances obtenues sous contrôle électronique du faisceau lumineux selon l'invention, après optimisation éventuelle de ses propres paramètres (paramètres de fréquence et de rapport cyclique permettant à l'utilisateur d'obtenir un meilleur confort de lecture).

### 4. Liste des figures.

L'invention sera mieux comprise, et d'autres particularités et avantages apparaîtront à la lecture de la description qui va suivre, la description faisant référence aux dessins annexés parmi lesquels :
- la **figure 1** est un schéma de représentation d'un signal EL de commande (contrôle) d'un module d'éclairage selon un mode de réalisation particulier et non limitatif de l'invention.
- la **figure 2** est une représentation structurelle de l'architecture d'un dispositif d'éclairage LIGHT selon un mode de réalisation particulier et non-limitatif de l'invention.

### 5. Description détaillée de modes de réalisation de l'invention.

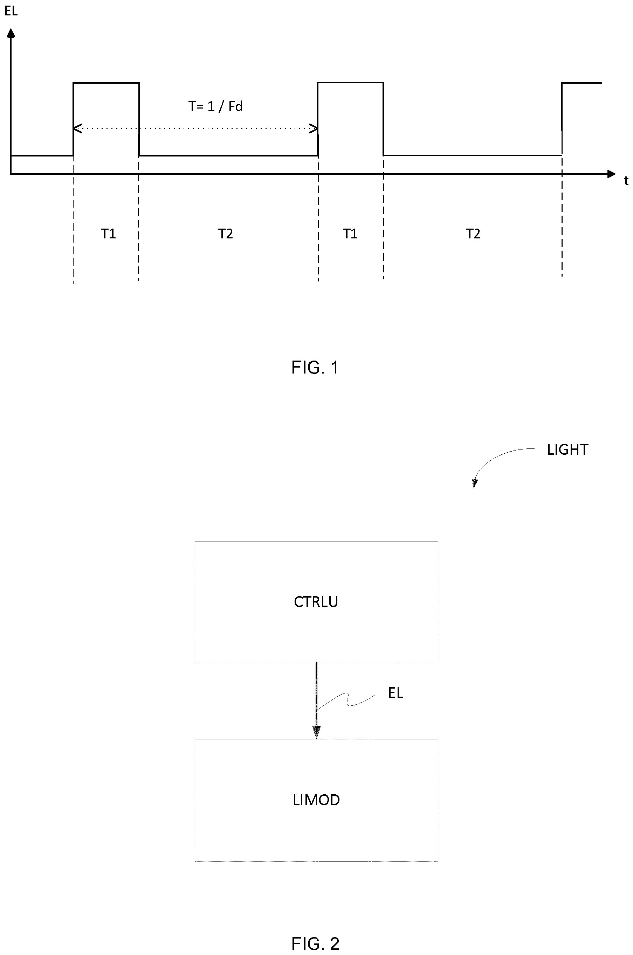

Sur la **figure 2****,** les modules représentés sont des unités fonctionnelles, qui correspondent ou non à des unités physiquement distinguables. Par exemple, ces modules ou certains d'entre eux sont regroupés dans un unique composant. *A contrario,* selon d'autres modes de réalisation, certains modules sont composés d'entités physiques séparées.

La **figure 1** est une représentation temporelle d'un signal EL de commande d'activation et de désactivation (suppression) d'un faisceau d'éclairage dans le spectre de la lumière visible, dans un dispositif d'éclairage LIGHT, selon un mode de réalisation particulier et non limitatif de l'invention. Le signal EL varie selon le temps t et prend périodiquement deux états successifs. Selon le mode de réalisation préféré, une assertion du signal EL à l'état haut contrôle une activation du faisceau d'éclairage du dispositif d'éclairage LIGHT susceptible d'éclairer ainsi, de manière discontinue un contenu graphique et/ou textuel sur un support quelconque, tel qu'un livre, par exemple. Le signal EL désactive (supprime ou inhibe) le faisceau lumineux du module d'éclairage lorsqu'il est positionné à l'état bas. Le signal EL est un signal périodique de fréquence prédéterminé Fd tel que Fd = 1/(T1+T2). T1 est la période d'activation du faisceau lumineux dans le spectre de la lumière visible, soit la période d'éclairage d'un support portant un motif représentant un contenu graphique et/ou textuel vers lequel le faisceau est orienté. T2 est la période, dite période de désactivation, durant laquelle le faisceau est supprimé ou rendu inactif, ou en d'autres termes la période pendant laquelle le contenu graphique et/ou textuel sur un support vers lequel le faisceau est orienté n'est plus éclairé par le faisceau. Les termes "contenu graphique" sont à interpréter ici comme n'importe quel contenu représenté sur un support quelconque, notamment au format papier mais pas seulement, et constitué d'éléments élémentaires tels que, à titre d'exemple des points ou pixels juxtaposés, de sorte que le contenu représente des éléments de formes variées et notamment un ou plusieurs contenus textuels construits à partir de signes ou de symboles d'un ou plusieurs alphabets.

Ainsi un contenu textuel apposé sur un support correspond ici à un contenu interprétable dans un ou plusieurs langages, susceptible d'être lu et interprété par un sujet, utilisateur du dispositif d'éclairage, positionné de sorte à regarder le support ainsi éclairé pour une opération de lecture ou de visualisation. Un tel dispositif est, à titre d'exemple, une lampe de bureau, une lampe torche, une lampe frontale, un stylo-lampe, une lampe de poche, une lampe de téléphone, une lampe de smartphone, une montre-lampe, une lampe de porte clef, un plafonnier, un lampadaire, une ampoule, un tube d'éclairage, un projecteur, un projecteur d'automobile, un rétroprojecteur, un revêtement mural lumineux, un tapis lumineux, un bracelet lumineux, un cadre lumineux, une mini-lampe à positionner sur un livre par le biais d'un système d'attache, un dispositif configuré pour l'éclairage intérieur (d'un local) ou encore un dispositif configuré pour un éclairage extérieur, urbain ou non. Cette liste d'exemples n'étant pas exhaustive. Selon le mode de réalisation préféré de l'invention, le rapport cyclique T1/(T1+T2) entre les périodes d'activation et de désactivation (ou inhibition) du faisceau lumineux, respectivement de durées T1 et T2, a une valeur comprise entre 15% et 30% du cycle, et la fréquence Fd de variation du signal EL est comprise entre 60 Hz et 90 Hz.

De façon préférée, la fréquence du signal est égale à 70 Hz ou 84 Hz et le rapport cyclique T1 / (T1+T2) est égal à 20%.

Avantageusement, le signal de contrôle EL peut être aisément forcé de façon prolongée dans son état associé à une activation du faisceau lumineux, ce qui correspond à un débrayage de la méthode d'éclairage mise en œuvre dans le dispositif LIGHT selon l'invention. Il serait ainsi possible de ne pas mettre en œuvre la méthode de contrôle d'un faisceau lumineux, dans un dispositif d'éclairage selon l'invention, dans le cas où, pour un sujet non dyslexique, une gêne visuelle apparaitrait du fait de la discontinuité d'activation du faisceau d'éclairage.

Avantageusement, il est possible d'affiner le réglage de la fréquence Fd dans l'intervalle de valeurs décrit dans le but d'adapter la période T à la sensibilité d'un utilisateur du dispositif d'éclairage LIGHT, dans la gamme de fréquences indiquée. En effet, chaque individu possède une sensibilité propre en termes de vision et perçoit plus ou moins des variations de fréquence d'un faisceau lumineux. Ainsi, un réglage fin peut être rendu accessible à l'utilisateur par l'intermédiaire d'un bouton de réglage, d'un curseur, implémenté matériellement ou via une interface utilisateur quelconque (éléments graphiques d'un menu sur un écran de contrôle, par exemple).

La **figure 2** est une représentation structurelle d'un dispositif d'éclairage LIGHT selon un mode de réalisation particulier et non-limitatif de l'invention. Cette figure représente l'architecture globale du dispositif d'éclairage LIGHT, encore communément appelé « lampe ». Le dispositif LIGHT comprend deux modules principaux qui sont une unité de contrôle CTRLU et un module d'éclairage LIMOD. L'unité de contrôle CTRLU est le cœur du système en termes de contrôle et comprend un circuit bistable (ou hacheur) classique, adapté à la génération du signal EL. Le circuit de hachage bistable de l'unité de contrôle CTRLU délivre le signal EL caractérisé par la fréquence Fd et par son rapport cyclique T1/(T1+T2). Bien évidemment l'unité de contrôle CTRLU comprend tous les éléments usuels mis en œuvre dans une telle architecture, tels que, à titre d'exemples, un ou plusieurs amplificateurs opérationnels, des résistances et capacités, une ou plusieurs diodes, une alimentation, un circuit de remise à zéro, un circuit de supervision d'alimentation, une interface de puissance, un amplificateur de courant, la liste de ces éléments n'est pas exhaustive. Les détails architecturaux de l'unité de contrôle CTRLU ne sont pas décrits plus encore dans la mesure où ceux-ci ne sont pas utiles à la compréhension de l'invention. Selon un mode de réalisation de l'invention, le module CTRLU comprend un circuit bistable construit autour d'un amplificateur opérationnel, couplé à un circuit d'amplification de courant. Avantageusement, l'utilisation d'un amplificateur de courant permet d'obtenir une énergie moyenne suffisante à un éclairage correct bien que le faisceau lumineux ainsi généré soit discontinu. Le module d'éclairage LIMOD est un module d'éclairage adapté à la génération d'un faisceau lumineux dans le spectre de la lumière visible, ou sensiblement plus large. Avantageusement, le faisceau peut être plus ou moins focalisé pour être alors configuré pour l'éclairage d'une surface plus ou moins grande. Une telle focalisation peut être réalisée par l'utilisation d'éléments optiques (lentilles) ou encore d'éléments mécaniques (diaphragmes), ou les deux à la fois.

Avantageusement, le faisceau lumineux est réalisé à partir d'un ou plusieurs objets électroluminescents de type LED, de l'acronyme anglais « Light-Emitting Diode » et qui signifie « diode à émission de lumière ». Bien évidemment, le faisceau lumineux peut être réalisé à partir d'autres éléments lumineux, dès lors que la désactivation du faisceau peut-être suffisamment rapide pour respecter les cycles comprenant des périodes d'éclairage et d'inhibition du faisceau (périodes de non-éclairage).

Le terme « inhibition du faisceau » doit être interprété ici comme une disparition totale du faisceau ou un amoindrissement conséquent du niveau d'éclairage produit par le faisceau.

C'est la capacité à activer et inhiber successivement l'éclairage d'un support qui présente un ou plusieurs contenus graphiques et/ou textuels, sous contrôle du module bistable de l'unité CTRLU, qui permet avantageusement au cerveau d'un sujet de privilégier une image plutôt que son image miroir, perçue à partir du support lorsque ce dernier est éclairé par le dispositif d'éclairage LIGHT selon l'invention. Avantageusement, cela permet d'aider conséquemment la lecture et le déchiffrage de contenus textuels, chez un sujet présentant des troubles dyslexiques.

L'unité de contrôle CTRLU comprend en sortie un signal EL d'activation (ou d'extinction / inhibition) du faisceau d'éclairage, connecté en entrée du module d'éclairage LIMOD.

En d'autres termes, les variations du signal de contrôle du faisceau d'éclairage EL, opérées par l'unité de contrôle CTRLU comprenant un circuit bistable, à une fréquence Fd, permettent d'agir sur l'éclairage d'un contenu graphique représenté sur un support quelconque, de sorte que ce contenu graphique soit successivement éclairé puis moins (ou plus du tout) éclairé, périodiquement, sur le support, selon des cycles successifs de longueur totale T opérés à une fréquence Fd prédéterminée. Selon l'invention, les périodes d'éclairage successives T1 ont chacune une durée comprise dans un intervalle de valeurs allant de 15 à 30% de la durée T desdits cycles. La fréquence Fd des cycles (comprenant chacun une période d'activation du faisceau et une période de désactivation du faisceau) est comprise entre 60 et 90 Hz.

Selon un mode de réalisation, la fréquence Fd est fixe.

Selon un autre mode de réalisation, la fréquence Fd varie dans le temps.

Selon un mode de réalisation particulier, la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

Selon un mode de réalisation particulier, la vitesse de décroissance et égale à la vitesse de croissance.

Selon un mode de réalisation particulier, les pas successifs sont de durées égales.

Selon un autre mode de réalisation particulier, les pas successifs varient en durée de sorte que la valeur de ladite fréquence Fd évolue selon une forme d'onde en triangle, en scie, ou en sinusoïde entre ladite valeur maximale et ladite valeur minimale.

Avantageusement la durée T1 des périodes d'activation varie dans le temps en évoluant de façon continue ou discontinue entre des valeurs bornes allant de 15 à 30% de la durée T des cycles. Il est entendu ici par de « façon continue » une évolution par incrément des pas successifs de durées égales.

Le phénomène de wobulation ainsi créé et appliqué à la fréquence Fd prédéterminée (variation de la fréquence Fd) permet de balayer un grand nombre de fréquences entre 60 Hz et 90 Hz, dont certaines seront plus efficaces pour l'aide à la lecture. Ces fréquences plus efficaces varient selon le sujet dyslexique. En balayant toutes les fréquences entre 60 et 90 Hz, le dispositif de l'invention ne nécessite aucun réglage préalable et devient efficace pour un grand nombre d'utilisateurs. Ce phénomène de wobulation permet ainsi et dans certains cas, de réduire plus encore des ennuis liés à des troubles dyslexiques.

Un même avantage découle des variations de la durée T1 des périodes d'activation du faisceau lumineux.

Selon le mode de réalisation préféré de l'invention, le dispositif d'éclairage LIGHT est configuré pour générer un premier faisceau lumineux continu pendant toute sa durée d'utilisation et un dispositif mobile ou de filtrage, piloté ou mû, masque ou inhibe périodiquement le faisceau lumineux de sorte qu'un second faisceau soit généré avec des caractéristiques de fréquence et de rapport cycliques tels que ceux précédemment décrits (Fd comprise en 60 Hz et 90 Hz et T1/(T1+T2) entre 15 et 30 %. A titre d'exemples non-limitatifs, selon ce mode de réalisation, un premier faisceau peut être interrompu cycliquement par un élément rotatif présentant des surfaces pleines et des surfaces vides, ou encore des surfaces opaques et des surfaces translucides, adaptées, par leur rotation, à générer le second faisceau, dont l'effet est alors le même que celui décrit dans le premier mode de réalisation décrit précédemment.

L'invention ne se limite pas aux seuls modes de réalisation décrits ci-avant, mais s'applique à tout dispositif d'éclairage d'un contenu graphique et/ou textuel sur un support quelconque, mettant en œuvre des opérations successives d'activation d'un faisceau d'éclairage et d'inhibition de ce même faisceau, périodiquement, selon des cycles successifs opérés à une fréquence Fd prédéterminée entre 60 Hz et 90 Hz telle que les périodes d'éclairage T1 successives ont chacune une durée comprise dans un intervalle de valeurs allant de 15 à 30% de la durée T cycles opérés.

## Revendications

1. Dispositif d'éclairage (LIGHT) pour faciliter la lecture d'un contenu graphique et/ou textuel sur un support quelconque par des sujets dyslexiques, ledit dispositif d'éclairage comprenant une unité de contrôle (CTRLU) adaptée à la génération d'un signal (EL) de commande d'activation et de désactivation d'un faisceau lumineux non permanent et un module d'éclairage (LIMOD) adapté à la génération dudit faisceau lumineux non permanent dans un spectre de lumière visible, ledit dispositif d'éclairage (LIGHT) étant piloté par le signal (EL) de commande d'activation et de désactivation dudit faisceau lumineux non permanent, ledit dispositif d'éclairage (LIGHT) étant configuré pour activer et désactiver périodiquement ledit faisceau lumineux non permanent selon des cycles successifs opérés à une fréquence prédéterminée Fd qui est une réciproque de la somme d'une période d'activation dudit faisceau lumineux non permanent et d'une période de désactivation dudit faisceau lumineux non permanent, chaque cycle ayant une durée T et comprenant une période d'activation dudit faisceau lumineux non permanent de durée T1 suivie ou précédée d'une période de désactivation dudit faisceau lumineux non permanent de durée T2, la fréquence prédéterminée Fd étant tel que Fd = 1/(T1+T2), le signal (EL) de commande d'activation et de désactivation dudit faisceau lumineux non permanent étant **caractérisé par** la fréquence prédéterminée Fd et par son rapport cyclique T1/(T1 +T2), la fréquence prédéterminée Fd étant comprise dans un intervalle de valeurs allant de 60 à 90 Hz, et la durée T1 des périodes d'activation dudit faisceau lumineux non permanent étant comprise dans un intervalle de valeurs allant de 15 à 30% de la durée T des cycles, ledit dispositif étant **caractérisé en ce qu'**il comprend un commutateur configuré pour obtenir sélectivement un faisceau lumineux permanent dit éclairage ordinaire et ledit faisceau lumineux non permanent sous contrôle électronique, afin de permettre à un utilisateur sujet à des troubles dyslexiques de comparer ses performances usuelles en lecture obtenues avec l'éclairage ordinaire à ses performances obtenues sous contrôle électronique dudit faisceau lumineux non permanent.

2. Dispositif d'éclairage selon la revendication 1, selon lequel l'unité de contrôle (CTRLU) est configurée pour varier selon le temps le signal (EL) de commande d'activation et de désactivation dudit faisceau lumineux non permanent en prenant successivement un état haut et un état bas, ledit faisceau lumineux non permanent étant désactivé à l'état bas et étant activé à l'état haut.

3. Dispositif d'éclairage selon la revendication 2, **caractérisé en ce que** l'unité de contrôle (CTRLU) est configurée pour forcer ledit signal (EL) de commande d'activation et de désactivation dudit faisceau lumineux non permanent de façon prolongée dans un état associé à une activation dudit faisceau lumineux non permanent, de manière à obtenir un débrayage de l'éclairage mis en œuvre par ledit dispositif d'éclairage.

4. Dispositif d'éclairage selon l'une quelconque des revendications 1 à 3, dans lequel ladite la fréquence prédéterminée Fd est égale à 70 Hz ou 84 Hz et le rapport cyclique T1 / (T1+T2) est égal à 20%.

5. Dispositif d'éclairage selon la revendication 1 à 4, dans lequel ledit dispositif d'éclairage (LIGHT) est configuré pour varier ladite fréquence prédéterminée Fd dans le temps.

6. Dispositif d'éclairage selon la revendication 4, dans lequel ladite fréquence prédéterminée Fd est dite de wobulation, mettant à profit les mécanismes hébbiens des neurones du cortex et le dispositif d'éclairage (LIGHT) est configuré pour varier ladite fréquence prédéterminée Fd en croissant par pas successifs jusqu'à une valeur maximale selon une première vitesse, dite vitesse de croissance, puis en décroissant par pas successifs jusqu'à une valeur minimale selon une seconde vitesse, dite vitesse de décroissance, les variations croissantes et décroissantes étant répétées itérativement dans le temps.

7. Dispositif d'éclairage selon la revendication 6, dans lequel ladite vitesse de décroissance est égale à ladite vitesse de croissance.

8. Dispositif d'éclairage selon la revendication 6, dans lequel lesdits pas successifs sont de durées égales.

9. Dispositif d'éclairage selon la revendication 6, dans lequel le dispositif d'éclairage (LIGHT) est configuré pour varier lesdits pas successifs en durée de sorte que la valeur de ladite fréquence prédéterminée Fd évolue selon une forme d'onde en triangle, en scie, ou en sinusoïde entre ladite valeur maximale et ladite valeur minimale.

10. Dispositif d'éclairage selon l'une quelconque des revendications précédentes, dans lequel le dispositif d'éclairage (LIGHT) est configuré pour varier la durée T1 desdites périodes d'activation dudit faisceau lumineux non permanent dans le temps.

11. Dispositif d'éclairage selon l'une quelconque des revendications précédentes, dans lequel ledit faisceau lumineux non permanent est généré à partir d'un ou plusieurs éléments de type LED.

12. Dispositif d'éclairage selon l'une quelconque des revendications précédentes, **caractérisé en ce qu'**il est d'un type compris dans la liste : une lampe de bureau, une lampe torche, une lampe frontale, un stylo-lampe, une lampe de poche, une lampe de téléphone, une lampe de smartphone, une montre-lampe, une lampe de porte clef, un plafonnier, un lampadaire, une ampoule, un tube d'éclairage, un projecteur, un projecteur d'automobile, un rétroprojecteur, un revêtement mural lumineux, un tapis lumineux, un bracelet lumineux, un cadre lumineux, une mini-lampe à positionner sur un livre, un dispositif configuré pour l'éclairage intérieur ou encore un dispositif configuré pour un éclairage extérieur, urbain ou non.

13. Dispositif d'éclairage selon l'une quelconque des revendications précédentes, **caractérisé en ce qu'**il s'agit d'une lampe de bureau ou d'une mini-lampe à positionner sur un livre.

14. Procédé pour faciliter la lecture d'un contenu graphique et/ou textuel sur un support quelconque par un sujet dyslexique, **caractérisé en ce qu'**il utilise un dispositif d'éclairage tel que défini selon l'une quelconque des revendications précédentes.

15. Procédé pour faciliter la lecture d'un contenu graphique et/ou textuel selon la revendication 14, dans lequel ladite fréquence prédéterminée Fd est définie par un utilisateur dudit dispositif d'éclairage.

## Patentansprüche

1. Beleuchtungsvorrichtung (LIGHT) zur Leseerleichterung eines grafischen und/oder Textinhalts auf einem beliebigen Träger durch Legastheniker, wobei die Beleuchtungsvorrichtung eine Steuereinheit (CTRLU) umfasst, die zur Erzeugung eines Aktivierungs- und Deaktivierungssteuersignals (EL) eines nicht permanenten Lichtstrahls und eines Beleuchtungsmoduls (LIMOD), das zur Erzeugung des nicht permanenten Lichtstrahls in einem Spektrum sichtbaren Lichts angepasst ist, wobei die Beleuchtungsvorrichtung (LIGHT) durch das Aktivierungs- und Deaktivierungssteuersignal (EL) des nicht permanenten Lichtstrahls gesteuert ist,
wobei die Beleuchtungsvorrichtung (LIGHT) dazu konfiguriert ist, periodisch den nicht permanenten Lichtstrahl gemäß aufeinanderfolgenden Zyklen zu aktivieren und zu deaktivieren, die mit einer vorbestimmten Frequenz Fd betrieben werden, die ein Kehrwert der Summe einer Aktivierungsperiode des nicht permanenten Lichtstrahls und einer Deaktivierungsperiode des nicht permanenten Lichtstrahls ist,
wobei jeder Zyklus eine Dauer T aufweist und eine Aktivierungsperiode des nicht permanenten Lichtstrahls mit einer Dauer T1 umfasst, der eine Deaktivierungsperiode des nicht permanenten Lichtstrahls mit einer Dauer T2 vorausgeht oder folgt, wobei die vorbestimmte Frequenz Fd derart ist, dass Fd = 1/(T1+T2), wobei das Aktivierungs- und Deaktivierungssteuersignal (EL) des nicht permanenten Lichtstrahls durch die vorbestimmte Frequenz Fd und ihr zyklisches Verhältnis T1/(T1+T2) gekennzeichnet ist, wobei die vorbestimmte Frequenz Fd in einem Intervall von Werten liegt, die von 60 bis 90 Hz reichen, und die Dauer T1 der Aktivierungsperioden des nicht permanenten Lichtstrahls in einem Bereich von Werten liegen, der von 15 bis 30 % der Dauer T der Zyklen reicht, wobei die Vorrichtung **dadurch gekennzeichnet ist, dass** sie einen Schalter umfasst, der dazu konfiguriert ist, selektiv einen permanenten Lichtstrahl, als gewöhnliche Beleuchtung bezeichnet, und den nicht permanenten Lichtstrahl unter elektronischer Steuerung zu erhalten, um es einem Benutzer, der unter Legasthenie leidet, zu erlauben, seine üblichen Leseleistungen, die mit gewöhnlicher Beleuchtung erhalten werden, mit seinen Leistungen zu vergleichen, die unter elektronischer Steuerung des nicht permanenten Lichtstrahls erhalten werden.

2. Beleuchtungsvorrichtung nach Anspruch 1, wobei die Steuereinheit (CTRLU) dazu konfiguriert ist, das Aktivierungs- und Deaktivierungssteuersignal (EL) des nicht permanenten Lichtstrahls durch aufeinander folgendes Annehmen eines hohen und eines niedrigen Zustands mit der Zeit zu variieren, wobei der nicht permanente Lichtstrahl im niedrigen Zustand deaktiviert und im hohen Zustand aktiviert ist.

3. Beleuchtungsvorrichtung nach Anspruch 2, **dadurch gekennzeichnet, dass** die Steuereinheit (CTRLU) dazu konfiguriert ist, das Aktivierungs- und Deaktivierungssteuersignal (EL) des nicht permanenten Lichtstrahls auf verlängerte Art in einen Zustand zu zwingen, der einer Aktivierung des nicht permanenten Lichtstrahls zugeordnet ist, um ein Entkoppeln der Beleuchtung, die von der Beleuchtungsvorrichtung umgesetzt wird, zu erhalten.

4. Beleuchtungsvorrichtung nach einem der Ansprüche 1 bis 3, wobei die vorbestimmte Frequenz Fd gleich 70 Hz oder 84 Hz ist, und das zyklische Verhältnis T1/(T1+T2) gleich 20 % ist.

5. Beleuchtungsvorrichtung nach Anspruch 1 bis 4, wobei die Beleuchtungsvorrichtung (LIGHT) dazu konfiguriert ist, die vorbestimmte Frequenz Fd mit der Zeit zu variieren.

6. Beleuchtungsvorrichtung nach Anspruch 4, wobei die vorbestimmte Frequenz Fd als Wobbelfrequenz bezeichnet wird, die die Hebbel-Mechanismen der Neuronen der Hirnrinde nutzt, und die Beleuchtungsvorrichtung (LIGHT) dazu konfiguriert ist, die vorbestimmte Frequenz Fd zu variieren, indem sie in aufeinanderfolgenden Schritten bis zu einem Höchstwert gemäß einer ersten Geschwindigkeit, der sogenannten Wachstumsgeschwindigkeit, ansteigt und dann in aufeinanderfolgenden Schritten bis zu einem Minimalwert gemäß einer zweiten Geschwindigkeit, der sogenannten Abklinggeschwindigkeit, abfällt, wobei die ansteigenden und abfallenden Variationen iterativ mit der Zeit wiederholt werden.

7. Beleuchtungsvorrichtung nach Anspruch 6, wobei die Abklinggeschwindigkeit gleich der Wachstumsgeschwindigkeit ist.

8. Beleuchtungsvorrichtung nach Anspruch 6, wobei die aufeinanderfolgenden Schritte gleiche Dauern aufweisen.

9. Beleuchtungsvorrichtung nach Anspruch 6, wobei die Beleuchtungsvorrichtung (LIGHT) dazu konfiguriert ist, die aufeinanderfolgenden Schritte hinsichtlich der Dauer derart zu variieren, dass sich der Wert der vorbestimmten Frequenz Fd gemäß einer Dreiecks-, Säge- oder Sinuswellenform zwischen dem Höchstwert und dem Minimalwert entwickelt.

10. Beleuchtungsvorrichtung nach einem der vorstehenden Ansprüche, wobei die Beleuchtungsvorrichtung (LIGHT) dazu konfiguriert ist, die Dauer T1 der Aktivierungsperioden des nicht permanenten Lichtstrahls mit der Zeit zu variieren.

11. Beleuchtungsvorrichtung nach einem der vorstehenden Ansprüche, wobei der nicht permanente Lichtstrahl ausgehend von einem oder mehreren Elementen vom Typ LED erzeugt wird.

12. Beleuchtungsvorrichtung nach einem der vorstehenden Ansprüche, **dadurch gekennzeichnet, dass** sie von einem Typ ist, der in der folgenden Liste enthalten ist: eine Bürolampe, eine Stablampe, eine Stirnlampe, eine Stiftlampe, eine Taschenlampe, eine Telefonlampe, eine Smartphone-Lampe, eine Uhr-Lampe, eine Schlüsselanhängerlampe, eine Deckenleuchte, eine Stehlampe, eine Glühbirne, ein Leuchtrohr, ein Projektor, ein Autoscheinwerfer, ein Overheadprojektor, eine leuchtende Wandverkleidung, ein Leuchtteppich, ein Leuchtarmband, ein Leuchtrahmen, eine Minilampe, die auf einem Buch positioniert wird, eine Vorrichtung, die für die Innenbeleuchtung konfiguriert ist, oder auch eine Vorrichtung, die für eine Außenbeleuchtung, städtisch oder nicht städtisch, konfiguriert ist.

13. Beleuchtungsvorrichtung nach einem der vorstehenden Ansprüche, **dadurch gekennzeichnet, dass** es sich um eine Bürolampe oder eine auf einem Buch zu positionierende Minilampe handelt.

14. Verfahren zur Leseerleichterung eines grafischen und/oder Textinhalts auf einem beliebigen Träger durch einen Legastheniker, **dadurch gekennzeichnet, dass** es eine Beleuchtungsvorrichtung nach einem der vorstehenden Ansprüche umfasst.

15. Verfahren zum Erleichtern des Lesens eines grafischen und/oder Textinhalts nach Anspruch 14, wobei die vorbestimmte Frequenz Fd durch einen Benutzer der Beleuchtungsvorrichtung festgelegt wird.

## Claims

1. A lighting device (LIGHT) for facilitating the reading of graphic and/or text content on any support by dyslexic subjects, said lighting device comprising a control unit (CTRLU) suitable for the generation of an activation and deactivation command signal (EL) of a non-permanent light beam and a lighting module (LIMOD) suitable for the generation of said non-permanent light beam in a visible light spectrum, said lighting device (LIGHT) being controlled by the activation and deactivation command signal (EL) of said non-permanent light beam,
said lighting device (LIGHT) being configured to periodically activate and deactivate said non-permanent light beam according to successive cycles operated at a predetermined frequency Fd which is a reciprocal of the sum of an activation period of said non-permanent light beam and of a deactivation period of said non-permanent light beam,
each cycle having a duration T and comprising a period of activation of said non-permanent light beam of duration T1 followed or preceded by a period of deactivation of said non-permanent light beam of duration T2, the predetermined frequency Fd being such that Fd=1/(T1+T2), the activation and deactivation command signal (EL) of said non-permanent light beam being **characterized by** the predetermined frequency Fd and the cyclic ratio thereof T1/(T1+T2), the predetermined frequency Fd being comprised in an interval of values going from 60 to 90 Hz, and a duration T1 of the activation periods of said non-permanent light beam being comprised in an interval going from 15 to 30% of the duration T of the cycles, said device being **characterized in that** same comprises a switch configured for selectively obtaining a permanent light beam called ordinary lighting and said non-permanent light beam under electronic control, so as to allow a user subject to dyslexic problems to compare their usual performance in reading with ordinary lighting to their performance obtained under electronic control of said non-permanent light beam.

2. The lighting device according to claim 1, according to which the control unit (CTRLU) is configured for varying according to time, the activation and deactivation command signal (EL) of said non-permanent light beam taking successively a high state and a low state, said non-permanent light beam being deactivated in the low state and being activated in the high state.

3. The lighting device according to claim 2, **characterized in that** the control unit (CTRLU) is configured for forcing said activation and deactivation command signal (EL) of said non-permanent light beam, in a prolonged manner, into a state associated with an activation of said non-permanent light beam, so as to obtain a disconnection of the lighting implemented by said lighting device.

4. The lighting device according to any of claims 1 to 3, wherein said predetermined frequency Fd is equal to 70 Hz or 84 Hz and the cyclic ratio T1/(T1+T2) is equal to 20%.

5. The lighting device according to claim 1 to 4, wherein said lighting device (LIGHT) is configured for varying said predetermined frequency Fd over time.

6. The lighting device according to claim 4 wherein said predetermined frequency Fd is called a wobbling frequency, drawing on the Hebbian mechanisms of neurons of the cortex and the lighting device (LIGHT) is configured for varying said predetermined frequency Fd increasing by successive steps up to a maximum value according to a first speed, called speed of increase, then decreasing by successive steps down to a minimum value according to a second speed, called speed of decrease, the increasing and decreasing changes being repeated iteratively over time.

7. The lighting device according to claim 6, wherein said speed of decrease is equal to said speed of increase.

8. The lighting device according to claim 6, wherein said successive steps have equal durations.

9. The lighting device according to claim 6, wherein the lighting device (LIGHT) is configured for varying said successive steps in duration so that the value of said predetermined frequency Fd changes according to a triangular, sawtooth or sinusoidal wave form between said maximum value and said minimum value.

10. Th lighting device according to any of the preceding claims wherein the lighting device (LIGHT) is configured to vary the duration T1 of said periods of activation of said non-permanent light beam over time.

11. The lighting device according to any of the preceding claims, wherein said non-permanent light beam is generated by one or a plurality of LED type elements.

12. The lighting device according to any of the preceding claims, **characterized in that** same is of a type comprised in the list: a desk lamp, a torch, a frontal lamp, a penlight, a pocket lamp, a telephone lamp, a smartphone lamp, a watch lamp, a key-ring lamp, a ceiling light, a floor lamp, a bulb, a lighting tube, a projector, a car headlight, an overhead projector, a luminous wall covering, a luminous mat, a luminous bracelet, a luminous frame, a mini-reading light to be positioned on a book, a device configured for interior lighting or else a device configured for outside lighting, whether urban or not.

13. The lighting device according to any of the preceding claims, **characterized in that** same is a desk lamp or a mini-reading light to be positioned on a book.

14. A method for facilitating the reading of a graphic and/or text content on any substrate by a dyslexic subject, **characterized in that** said method uses a lighting device such as defined in any of the preceding claims.

15. The method for facilitating the reading of a graphic and/or a text content according to claim 14 wherein said predetermined frequency Fd is defined by the user of said lighting device.
